# EUROPEAN PATENT APPLICATION

(11) **EP 4 631 953 A1**
(43) Date of publication of application: **15.10.2025**
(21) Application number: 23900720.6
(22) Date of filing: 07.12.2023
(51) Int. Cl.: C07K 1/18

(54) **METHOD FOR ISOLATING GLYCOPEPTIDE**

(30) Priority: 07.12.2022 JP 2022195977
(71) Applicant: KH Neochem Co., Ltd., Tokyo 103-0022 (JP)
(72) Inventor: NAKAMACHI, Yuto, Kawasaki-shi, Kanagawa 212-0032 (JP)
(74) Representative: Mathys & Squire
(86) International application number: PCT/JP2023/043794
(87) International publication number: WO 2024/122608

(57) **Abstract**

The present invention relates to a method for separating, from a mixture containing glycoamino acids and one or more types of glycooligopeptides, the glycoamino acids and the glycooligopeptides by passing the mixture through an anion-exchange resin.

## Description

### Technical Field

The present invention relates to a method for separating glycopeptides.

### Background Art

Glycans are polymers in which monosaccharides are linked in a branching pattern, and their structures are diverse because the monosaccharides, which serve as basic bonding units, have multiple bonding points.

Glycans *in vivo* are present on proteins and cell surfaces, and are involved in protein and cell recognition as well as signaling.

It is also known that abnormalities in glycan structures are associated with diseases, such as cancer and abnormalities in protein quality control, and attempts are being made to use glycans to elucidate the disease mechanisms. In addition to elucidating the disease mechanisms, the application of glycans to diagnostic agents is also being attempted.

Furthermore, it is known that the function of glycans and the recognition of glycans by enzymes vary depending on the number of amino acids linked to the glycans. For further research, there is a need for a method to obtain multiple glycopeptides that have various peptide-forming amino acids and have different numbers of the amino acids.

Non-Patent Literature 1 discloses a method for purifying a glycoamino acid linked to a high-mannose-type glycan of 12 monosaccharides from delipidated egg yolk.

### Citation List

### Non-Patent Literature

Non-Patent Literature 1: Carbyhydrate Research (2015) vol. 411, pp. 37-41

### Summary of Invention

### Technical Problem

The problem to be solved by the present invention is to provide a method for separating, from a mixture containing glycoamino acids and one or more types of glycooligopeptides, the glycoamino acids and the glycooligopeptides.

### Solution to Problem

As a result of extensive research, the present inventors have found that an anion-exchange resin can be used to separate, from a mixture containing glycoamino acids and one or more types of glycooligopeptides, the glycoamino acids and the glycooligopeptides. Thus, the present invention has been completed.

Specifically, the present invention is as follows.
[1] A method for separating, from a mixture containing glycoamino acids and one or more types of glycooligopeptides, the glycoamino acids and the glycooligopeptides by passing the mixture through an anion-exchange resin.
[2] The method according to [1], wherein the glycooligopeptides are glycodipeptides and/or glycotripeptides.
[3] The method according to [1] or [2], wherein the glycoamino acids and the glycooligopeptides are each derived from an antibody.
[4] The method according to any one of [1] to [3], wherein the glycoamino acids and the glycooligopeptides each have a glycan of 8 to 12 monosaccharides.
[5] The method according to any one of [1] to [4], wherein the glycoamino acids and the glycooligopeptides each have a glycan of only neutral monosaccharides.

### Advantageous Effect of Invention

According to the present invention, it is possible to provide a method for separating, from a mixture containing glycoamino acids and one or more types of glycooligopeptides, the glycoamino acids and the glycooligopeptides.

### Brief Description of Drawings

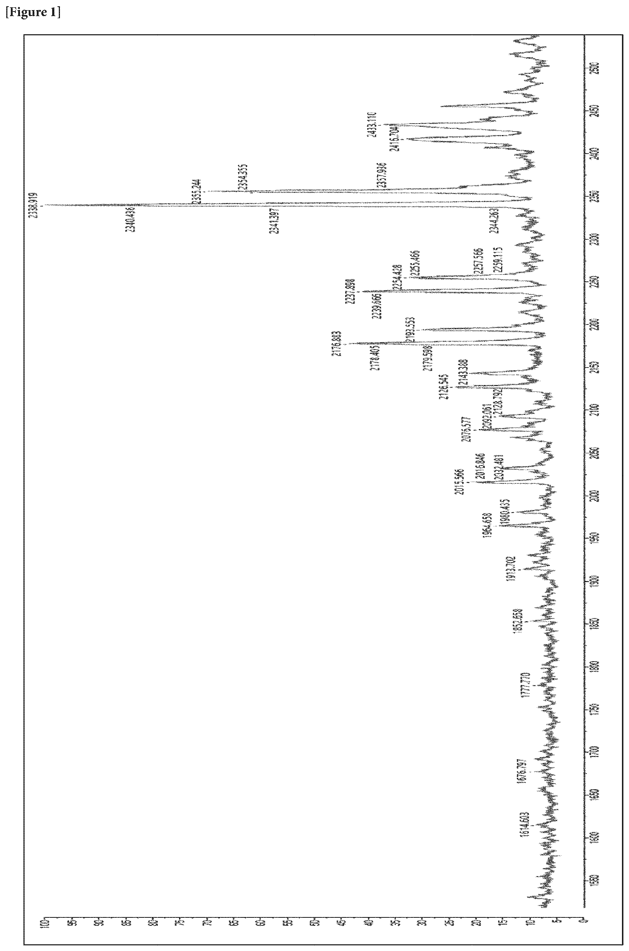

[Figure 1] Figure 1 shows the results of mass spectrometry of a mixture containing multiple glycopeptides by MALDI-TOF MS. It was indicated that the glycopeptides contained in the mixture each had a peptide of 1 to 3 amino acids and had a glycan of only 8 to 12 neutral monosaccharides or a complex-type glycan of 9 to 11 monosaccharides. In the graph, the horizontal axis represents the values of "m/z," and the vertical axis represents the values of "% intensity."

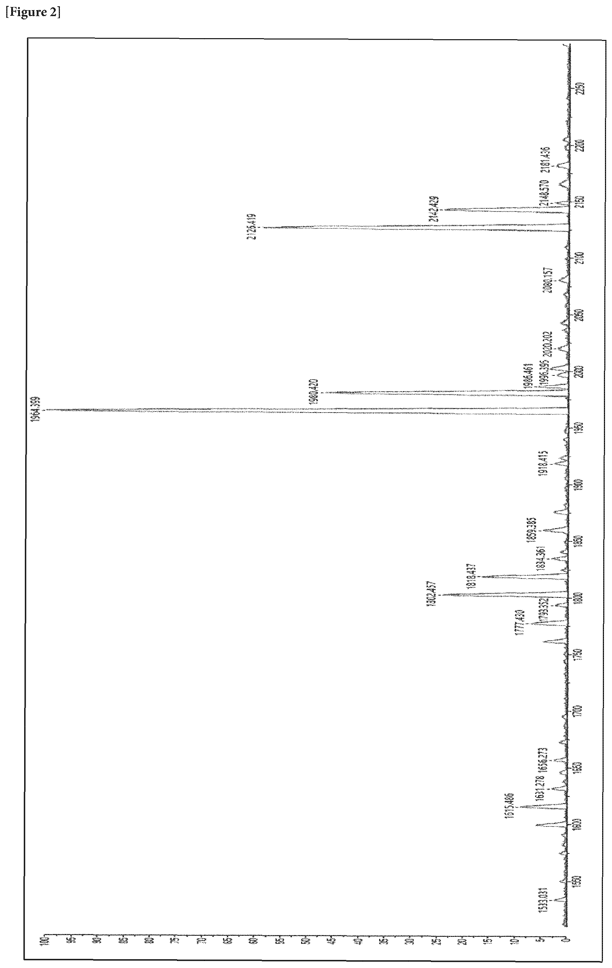

[Figure 2] Figure 2 shows the results of mass spectrometry of glycopeptides contained in a fraction eluted in about 300 to 500 mL by anion-exchange chromatography. It was indicated that the glycopeptides contained in the fraction had a peptide of 1 amino acid and had a glycan of only 8 to 12 neutral monosaccharides or a complex-type glycan of 9 to 11 monosaccharides. In the graph, the horizontal axis represents the values of "m/z," and the vertical axis represents the values of "% intensity."

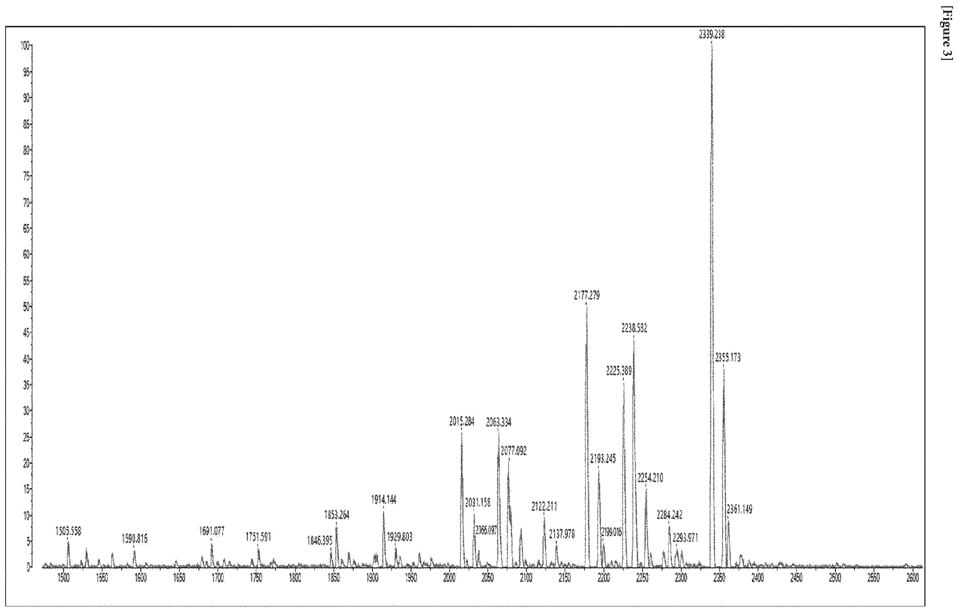

[Figure 3] Figure 3 shows the results of mass spectrometry of glycopeptides contained in a fraction eluted in about 800 to 1000 mL by anion-exchange chromatography. It was indicated that the glycopeptides contained in the fraction had a peptide of 2 or 3 amino acids and had a glycan of only 8 to 12 neutral monosaccharides. In the graph, the horizontal axis represents the values of "m/z," and the vertical axis represents the values of "% intensity."

### Description of Embodiments

The embodiment for carrying out the present invention will be described in detail below. The present invention is not limited to the following embodiment, and various modifications can be made within the scope of the gist thereof.

The method for separating glycopeptides in the present embodiment is a method for separating, from a mixture containing glycoamino acids and one or more types of glycooligopeptides, the glycoamino acids and the glycooligopeptides by passing the mixture through an anion-exchange resin. In the present specification, glycoamino acids and glycooligopeptides may be collectively referred to as glycopeptides.

### (Peptides of Glycopeptides)

In the present embodiment, the glycopeptide is one in which a glycan and a peptide are covalently linked together. In this paragraph, the peptide may mean an amino acid.

In the glycopeptide, a glycan is linked to any amino acid residue present in the peptide. The amino acid residue to which the glycan is linked is not particularly limited; however, examples thereof in a glycopeptide that is also referred to as an N-glycopeptide include asparagine (Asn), and examples thereof in a glycopeptide that is also referred to as an O-glycopeptide include threonine (Thr) and serine (Ser).

In the N-glycopeptide, a glycan is linked to the nitrogen atom of the side-chain CONH₂ group of Asn. In the O-glycopeptide, glycans are linked to the oxygen atoms of the side-chain hydroxyl groups of Thr and Ser.

The binding of the glycan to the peptide may be a glycosidic bond at the 1-position of the reducing terminal in the glycan.

In the present embodiment, the glycoamino acid means a glycopeptide in which a glycan and one amino acid are linked together. The amino acid in the glycoamino acid may be any single amino acid, and is preferably Asn, Thr, or Ser, and more preferably Asn.

In the present embodiment, the glycooligopeptide means a glycopeptide in which a glycan and a peptide having 2 to 20 amino acid residues are linked together, and the amino acids in the peptide of the glycooligopeptide may be any amino acids. When the number of amino acids in the peptide of a glycooligopeptide is 2 to 5, the glycooligopeptide is also referred to as a glycodipeptide, a glycotripeptide, a glycotetrapeptide, or a glycopentapeptide, depending on the number of amino acids in the peptide to which glycans are linked. The glycooligopeptide is not particularly limited, but may be any combination of glycopeptides to which peptides having 2 to 20 amino acid residues are linked (but may be a single glycopeptide). The any combination of glycopeptides may be glycodipeptides, glycotripeptides, glycotetrapeptides, and/or glycopentapeptides, or may be glycodipeptides and/or glycotripeptides.

In the glycooligopeptide, the oligopeptide may be a single-stranded peptide in which amino acids, which are the building blocks of the oligopeptide, are linked by peptide bonds.

Although the oligopeptide of the glycooligopeptide is not particularly limited, a glycooligopeptide containing Asn, Thr, and/or Ser is preferred, and a glycooligopeptide containing Asn is more preferred. Asn, Thr, and Ser may be the N-terminal amino acids, the C-terminal amino acids, or amino acids located between the N-terminal and C-terminal amino acids, but are preferably the N-terminal amino acids.

In the present embodiment, when the glycan in the glycodipeptide is linked to Asn, Asn may be the N-terminal amino acid or the C-terminal amino acid, but is preferably the N-terminal amino acid. The other amino acid in the dipeptide of the glycodipeptide is preferably glycine (Gly). Asn and Gly each may be an L-amino acid or a D-amino acid, but are preferably L-amino acids. The dipeptide in the glycodipeptide preferably has a structure derived from a dipeptide represented by H₂N-Asn-Gly-COOH. Here, H₂N- is the α-amino group present in Asn, and -COOH is the carboxyl group present in Gly.

In the present embodiment, when the glycan in the glycotripeptide is linked to Asn, Asn may be the N-terminal amino acid, the C-terminal amino acid, or an amino acid located in the center, but is preferably the N-terminal amino acid. The tripeptide in the glycotripeptide preferably has a structure derived from a dipeptide represented by H₂N-Asn-X-COOH (X is any amino acid). In that case, when Asn is the N-terminal amino acid, the C-terminal amino acid is not particularly limited, but is preferably Thr or Ser. Asn, Gly, and Thr or Ser each may be an L-amino acid or a D-amino acid, but are preferably L-amino acids. The tripeptide in the glycotripeptide preferably has a structure derived from a tripeptide represented by H₂N-Asn-X-Thr/Ser-COOH (X is any amino acid). Here, H₂N- is the α-amino group present in Asn, and -COOH is the carboxyl group present in Thr or Ser.

When the tripeptide is represented by H₂N-Asn-X-Thr/Ser-COOH (X is any amino acid), X is preferably an amino acid other than proline, and more preferably Gly, although it is not particularly limited thereto.

In the present embodiment, when the glycan in the glycotetrapeptide is linked to Asn, Asn may be the N-terminal amino acid, the C-terminal amino acid, or an amino acid located at a position other than the N-terminus and C-terminus, but is preferably the N-terminal amino acid. The tetrapeptide in the glycotetrapeptide preferably has a structure represented by H₂N-Asn-X¹-X²-X³-COOH (X¹, X², and X³ are any amino acids). Here, X¹ is not particularly limited, but is preferably an amino acid other than proline, and more preferably Gly. X² is not particularly limited, but is preferably an amino acid other than proline, and more preferably Thr or Ser. X³ is not particularly limited, but is preferably any amino acid, and more preferably an amino acid other than proline. The tetrapeptide in the glycotetrapeptide is not particularly limited, but preferably has a structure represented by H₂N-Asn-Gly-Thr/Ser-X³-COOH (X³ is any amino acid). Here, H₂N- is the α-amino group present in Asn, and -COOH is the carboxyl group present in X³.

In the present embodiment, when the glycan in the glycopentapeptide is linked to Asn, Asn may be the N-terminal amino acid, the C-terminal amino acid, or an amino acid located at a position other than the N-terminus and C-terminus, but is preferably the N-terminal amino acid. The pentapeptide in the glycopentapeptide preferably has a structure represented by H₂N-Asn-X¹-X²-X³-X⁴-COOH (X¹, X², X³, and X⁴ are any amino acids). Here, X¹ is not particularly limited, but is preferably an amino acid other than proline, and more preferably Gly. X² is not particularly limited, but is preferably an amino acid other than proline, and more preferably Thr or Ser. X³ and X⁴ are not particularly limited, but are preferably any amino acids, and more preferably amino acids other than proline. The pentapeptide in the glycopentapeptide is not particularly limited, but particularly preferably has a structure represented by H₂N-Asn-Gly-Thr/Ser-X³-X⁴-COOH (X³ and X⁴ are any amino acids). Here, H₂N- is the α-amino group present in Asn, and -COOH is the carboxyl group present in X⁴.

The peptide of the glycooligopeptide preferably contains the amino acid of the glycoamino acid.

In this case, in the glycodipeptide, one amino acid may be added to the N-terminus or C-terminus of the amino acid of the glycoamino acid. In the glycotripeptide, two amino acids may be added to the N-terminus and/or C-terminus of the amino acid of the glycoamino acid, and one amino acid may be added to the N-terminus or C-terminus of the peptide of the glycodipeptide. In the glycotetrapeptide, three amino acids may be added to the N-terminus and/or C-terminus of the amino acid of the glycoamino acid, two amino acids may be added to the N-terminus and/or C-terminus of the peptide of the glycodipeptide, and one amino acid may be added to the N-terminus or C-terminus of the peptide of the glycotripeptide. In the glycopentapeptide, four amino acids may be added to the N-terminus and/or C-terminus of the amino acid of the glycoamino acid, three amino acids may be added to the N-terminus and/or C-terminus of the peptide of the glycodipeptide, two amino acids may be added to the N-terminus and/or C-terminus of the peptide of the glycotripeptide, and one amino acid may be added to the N-terminus and/or C-terminus of the peptide of the glycotetrapeptide.

In the present embodiment, in the glycoamino acids or glycooligopeptides contained in the mixture to be passed through an anion-exchange resin, one or more amino acid residues in each peptide may be protected.

When the peptide in the glycopeptide is protected, more specifically, when the amino acid of the glycoamino acid or one or more amino acids in the peptide of the glycooligopeptide are protected, being protected means that each amino acid is protected with a conventionally known protecting group for amino acids. For example, when the amino acid of a glycoamino acid is Asn, being protected means that Asn is protected, and such a glycoamino acid is also referred to as a glycoasparagine derivative. The glycoasparagine derivative may be contained in the glycoamino acid of the present invention. For example, when the peptide of a glycooligopeptide contains Asn, Gly, Thr or/and Ser, being protected means that one or more amino acids among Asn, Gly, Thr or/and Ser are protected. When one or more of the amino acids of a glycooligopeptide are protected, such a glycooligopeptide is also referred to as a glycooligopeptide derivative, and may be contained in the glycooligopeptides of the present invention.

In the case of L-Asn, Asn has the following structure. When a glycan is linked, the glycan is linked to the nitrogen atom in the CONH₂ group present in the side chain of the amino acid. For example, when Asn is the amino acid present at the N-terminus, the COOH group is amide-linked to the NH₂ group of another amino acid to form a peptide.

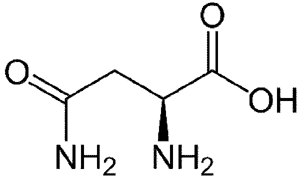

When Asn is the amino acid present at the N-terminus, the remaining NH₂ group can be present in a free state; thus, the amino group may be protected.

Gly has the following structure. When Gly is the amino acid present at the C-terminus, the NH₂ group is amide-linked to the COOH group of another amino acid.

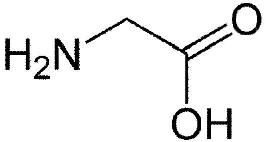

Therefore, the remaining COOH group can be present in a free state; thus, the carboxyl group may be protected.

In the case of L-Ser or L-Thr, Ser or Thr has the following structure. When Ser or Thr is the amino acid present at the C-terminus, the NH₂ group is amide-linked to the COOH group of another amino acid.

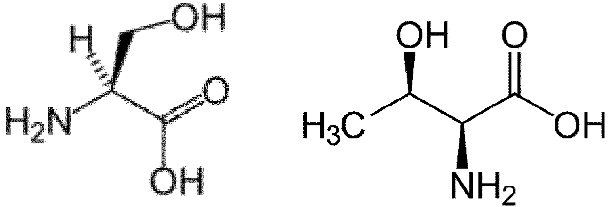

When Ser or Thr is the amino acid present at the C-terminus, the remaining COOH group can be present in a free state, and there is an OH group; thus, the carboxyl group and the hydroxyl group may be protected.

Known protecting groups can be used as the respective protecting groups for amino groups, carboxyl groups, and hydroxyl groups. For example, the protecting groups described in Greene's Protective Groups in Organic Synthesis may be selected.

As conventionally known protecting groups for amino acids that can be used as the protecting groups for amino groups and carboxyl groups, for example, the protecting groups described in Greene's Protective Groups in Organic Synthesis may be selected.

The protecting group for amino groups is not particularly limited, but examples thereof include an Fmoc group, a t-butoxycarbonyl (Boc) group, a benzyloxycarbonyl (Cbz or Z) group, a p-methoxybenzyloxycarbonyl (Z(OMe) or pMZ) group, a 2-(p-biphenyl)isopropyloxycarbonyl (Bpoc) group, and the like.

The protecting group for carboxyl groups is not particularly limited, but examples thereof include a methyl ester group, an ethyl ester group, a benzyl ester group, a t-butyl ester group, and the like. The methyl ester group, the ethyl ester group, the benzyl ester group, and the t-butyl ester group are protecting groups formed by ester formation between methyl alcohol, ethyl alcohol, benzyl alcohol, or t-butyl alcohol, and a carboxyl group, respectively.

Examples of the protecting group for hydroxyl groups include an acetyl (Ac) group, a benzoyl (Bz) group, a methoxymethyl (MOM) group, a benzyl (Bn) group, a t-butyl (t-Bu) group, and the like. The protecting group for hydroxyl groups may be a group known as a silyl-based protecting group, such as a trimethylsilyl (TMS) group, a triethylsilyl (TES) group, a t-butyldimethylsilyl (TBS) group, a triisopropylsilyl (TIPS) group, or a t-butyldiphenylsilyl (TBDPS) group.

The amino group and carboxyl group present in the glycopeptide, more specifically, the α-amino group in the N-terminal amino acid residue and the carboxyl group in the C-terminal amino acid residue of the peptide in the glycopeptide, may be present in a free state (NH₂ or COOH), may be present as NH₃⁺ or COO⁻, or may be a combination thereof.

The amino group and carboxyl group of the glycopeptide may be in the form of salts. When these groups are in the form of salts, examples thereof include, but are not particularly limited to, inorganic acids, such as hydrochloride, sulfate, and phosphate; and organic acids, such as acetate, propionate, tartrate, fumarate, maleate, malate, citrate, methanesulfonate, p-toluenesulfonate, and trifluoroacetate. For example, they may be alkali metal salts or alkaline earth metal salts, such as sodium salts, potassium salts, calcium salts, or magnesium salts.

### (Glycans of Glycopeptides)

In the present embodiment, the glycopeptides preferably have a glycan of 8 to 12 monosaccharides, although it is not particularly limited thereto.

The glycan has a structure in which monosaccharides, which are the building blocks of glycans, are linked by glycosidic bonds. The glycan may be branched, and generally has a non-reducing terminal and a reducing terminal.

Examples of the monosaccharides as the building blocks of glycans include, but are not particularly limited to, neutral monosaccharides and acidic monosaccharides. In the present embodiment, neutral monosaccharides are monosaccharides that do not have acidic groups such as carboxyl groups (including those in the form of salts or esters) as polar groups, and that have hydroxyl groups and/or amino groups (which may be modified with acyl groups etc.) as polar groups. Examples of neutral monosaccharides include, but are not particularly limited to, glucose (Glc), mannose (Man), galactose (Gal), xylose (Xyl), fucose (Fuc), N-acetylglucosamine (GlcNAc), N-acetylgalactosamine (GalNAc), and the like. In the present embodiment, acidic monosaccharides are monosaccharides that have acidic groups such as carboxyl groups (including those in the form of salts or esters) as polar groups. Examples of acidic monosaccharides include, but are not particularly limited to, N-acetylneuraminic acid (Neu5Ac), N-glycolylneuraminic acid (Neu5Gc), deaminoneuraminic acid (KDN), glucuronic acid (GlcA), iduronic acid (IdoA), and the like.

In the present embodiment, the glycans of the glycopeptides are not particularly limited, but are preferably N-glycans or O-glycans, and more preferably N-glycans.

The monosaccharides, which are the building blocks of glycans, each may be a D sugar or an L sugar. The bond between the monosaccharides is not particularly limited, but may be an α-bond or a β-bond. The position of the hydroxyl group of the reducing terminal sugar to which the non-reducing terminal sugar is linked in the bond between the monosaccharides is also not particularly limited, and the bond may be a 1→2 bond, a 1→3 bond, a 1→4 bond, a 1→6 bond, or a 2→6 bond.

Examples of the glycans of the glycopeptides include a glycan having only neutral monosaccharides, and a glycan having neutral monosaccharides and acidic monosaccharides. From the viewpoint of more efficiently separating glycoamino acids and glycooligopeptides, a glycan having only neutral monosaccharides is preferred.

The N-glycans or the O-glycans may be glycans having only neutral monosaccharides as building blocks, or glycans having neutral monosaccharides and acidic monosaccharides as building blocks. Furthermore, as the N-glycans, high-mannose-type glycans are known as glycans having only neutral monosaccharides, and hybrid-type and complex-type glycans are known as glycans having neutral monosaccharides and acidic monosaccharides. From the viewpoint of more efficiently separating glycoamino acids and glycooligopeptides, the Nglycans are preferably glycans having only neutral monosaccharides, and more preferably high-mannose-type glycans.

When the glycans of the glycopeptides are N-glycans, the reducing terminal GlcNAc of the glycans is linked to Asn, and glycopeptides in which GlcNAc is linked to Asn are also referred to as N-glycopeptides.

When the glycans of the glycopeptides are O-glycans, the sugar at the reducing terminal of the glycans is linked to Thr or Ser. Examples of the sugar at the reducing terminal of O-glycans include GlcNAc, GalNAc, Fuc, Man, Xyl, and the like.

The N-glycans or O-glycans may be naturally derived or artificially synthesized. The specific structure of the N-glycans or O-glycans is not particularly limited, but examples thereof include the glycans described in Nat Rev Nephrol. 2019 Jun; 15 (6): 346-366., and Organic Synthetic Chemistry, 1988, 46 (11), 1061-1072. In the present embodiment, the glycans of the glycoamino acids and glycooligosaccharides may be the same or different, and are preferably the same.

### (Glycopeptides Derived from an Antibody)

In the present embodiment, the glycopeptides are preferably glycopeptides derived from an antibody.

In the method for separating glycoamino acids and glycooligopeptides of the present embodiment, the origins of the glycoamino acids and glycooligopeptides may be the same or different; however, it is preferable that the glycoamino acids and the glycooligopeptides are derived from the same antibody. That is, the method is preferably a method for separating a mixture containing glycoamino acids and glycooligopeptides, both of which are derived from an antibody.

The antibody is not particularly limited, but examples thereof include avian-derived antibodies, human-derived antibodies, and the like. From the viewpoint of efficiently obtaining a large number of antibodies, avian-derived antibodies are preferred.

The avian antibody is not particularly limited and may be an antibody derived from avian species, and all of the amino acid sequences of the antibody are derived from avian species.

Avian species include chickens, goose, ducks, and the like, with chickens being preferred.

The isoform of the avian antibody is also not particularly limited, and examples thereof include IgY, IgA, and IgM, with IgY being preferred.

The avian antibody can be obtained from avian eggs or blood by a conventionally known method. The avian antibody is not particularly limited, but may be an egg-derived antibody. In particular, the avian antibody may be an antibody derived from eggs of chickens, goose, ducks, or the like. Since a large amount of IgY can be obtained from chicken eggs, an antibody derived from chicken eggs may be used.

Examples of glycans derived from antibodies include the glycans described in Glycobiology (2004) vol. 14, pp. 275-292.

IgY is explained as an example for the avian antibody. IgY contains Fab and Fc regions. The Fab region of the avian antibody is composed of heavy and light chains linked by disulfide bonds, and is linked to the Fc region via a hinge region.

In the present embodiment, when the glycoamino acids and the glycooligopeptides are derived from an avian antibody, since the glycoamino acids and the glycooligopeptides have glycans contained in the avian antibody, they may be glycoamino acids or glycooligopeptides derived from IgY, IgA, or IgM, or may be glycoamino acids or glycooligopeptides derived from antibody fragments, such as the Fab and Fc regions of IgY.

In the present embodiment, when the glycoamino acids or the glycooligopeptides are derived from the Fc region of an avian antibody, the structure of the peptide of the glycodipeptide or glycooligopeptides is not particularly limited as long as it is the structure of the glycan present in the Fc region of the avian antibody.

In the present embodiment, when the glycodipeptide is derived from the Fc region of an avian antibody, it is preferable that the glycodipeptide has a glycan of 8 to 12 monosaccharides, the dipeptide has Asn-Gly, and the glycodipeptide is an N-glycodipeptide in which the glycan is linked to Asn. In the glycodipeptide, one or more residues of Asn and Gly may be protected.

In the present embodiment, when the glycotripeptide is derived from the Fc region of an avian antibody, it is preferable that the glycotripeptide has a glycan of 8 to 12 monosaccharides, the tripeptide has Asn-Gly-Thr/Ser, and the glycotripeptide is an N-glycotripeptide in which the glycan is linked to Asn. In the glycotripeptide, one or more residues of Asn and Gly may be protected.

In the present embodiment, when the glycoamino acids or the glycooligopeptides are derived from the Fc region of an avian antibody, the structure of the glycan of the glycodipeptide or glycooligopeptides is not particularly limited as long as it is the structure of the glycan present in the Fc region of the avian antibody; however, a glycan of 8 to 12 monosaccharides is preferred.

The glycan of only neutral monosaccharides derived from IgY is not particularly limited, but examples thereof include the glycans shown in the following schematic diagram.

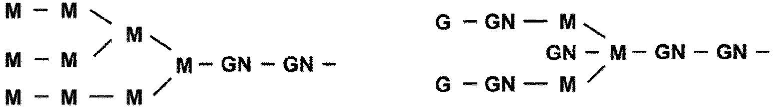

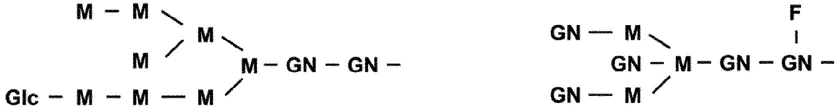

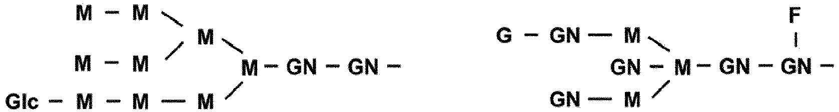

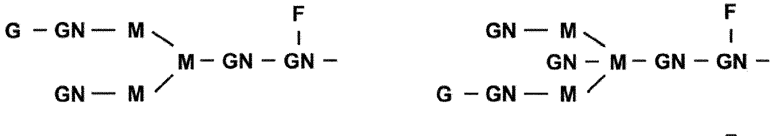

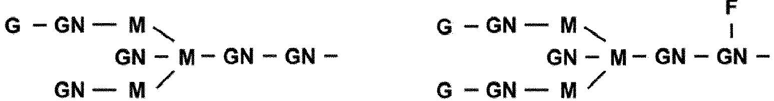

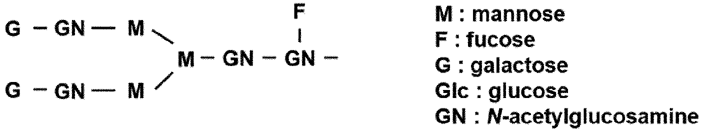

The right bond, to which the two GNs (N-acetylglucosamine, GlcNAc) are linked, is the bond to the peptide, and GlcNAc present on the right side forms the reducing terminal of the glycan.

The bond between the respective monosaccharides is not particularly limited as long as it is a bond present in the structure derived from IgY, and the bond may be an α-bond or a β-bond. The position of the hydroxyl group to which the glycan is linked is also not particularly limited as long as it is a position present in the structure derived from IgY, and the bond may be a 1→2 bond, a 1→3 bond, a 1→4 bond, a 1→6 bond, or a 2→6 bond. GlcNAc, glucose, mannose, and galactose are each generally a D sugar. Fucose is generally an L sugar.

### (Method for Separating Glycoamino Acids and One or more types of Glycooligopeptides)

The method of the present embodiment is a method for separating, from a mixture containing glycoamino acids and one or more types of glycooligopeptides, the glycoamino acids and the glycooligopeptides by passing the mixture through an anion-exchange resin.

By the separation method of the present embodiment, the glycoamino acids is obtained as one fraction, and the glycooligopeptides are obtained as one fraction. The glycoamino acids fraction may contain a mixture of glycoamino acids, and the glycooligopeptide fraction may contain a mixture of glycooligopeptides.

When the glycooligopeptides are glycodipeptides and/or glycotripeptides, the glycoamino acids and the glycodipeptides and/or glycotripeptides are separated.

Conventionally, it is difficult to separate glycoamino acids and glycooligopeptides; however, from the viewpoint that there is an even greater need to solve the problem by the present invention, the glycooligopeptides contained in the mixture are preferably glycodipeptides, glycotripeptides, glycotetrapeptides, and/or glycopentapeptides, and more preferably glycodipeptides and/or glycotripeptides.

### (Mixture Containing Glycoamino Acids and One or More types of Glycooligopeptides)

In the present embodiment, a mixture containing one or more types of glycooligopeptides means that the mixture contains glycooligopeptides having peptides with different numbers of amino acids. Although it is not particularly limited, for example, when the mixture contains glycodipeptides and glycotripeptides, it means that two types of glycooligopeptides are contained; when the mixture contains glycodipeptides, glycotripeptides, and glycotetrapeptides, and when the mixture contains glycodipeptides, glycotripeptides, and glycopentapeptides, it means that three type of glycooligopeptides are contained; and when the mixture contains glycodipeptides, glycotripeptides, glycotetrapeptides, and glycopentapeptides, it means that four types of glycooligopeptides are contained. The glycooligopeptides contained in the mixture are not particularly limited as long as their type is one or more; however, the number of types may be 2, 3, or 4, may be 1 to 4, may be 1 to 3, may be 1 or 2, or may be 2 or 3, 2 to 4, or 3 or 4. The mixture may also contain four or more types of glycooligopeptides, or two or more types of glycooligopeptides.

Even if glycopeptides differ in the structures of glycans, the amino acids in peptides, the binding modes between monosaccharides and peptides, and the like, they can be regarded as the same type of glycopeptide as long as the peptides have the same number of amino acids.

In the present embodiment, the mixture containing glycoamino acids and one or more types of glycooligopeptides can be prepared by a conventionally known method. Furthermore, the mixture containing glycoamino acids and one or more types of glycooligopeptides can also be prepared by mixing glycoamino acids or glycooligopeptides obtained by other methods.

Although it is not particularly limited, glycoamino acids or glycooligopeptides may be obtained by chemical synthesis, or may be obtained by cleavage from known glycoproteins other than antibodies.

The method using chemical synthesis is not particularly limited, but examples thereof include the methods described in Biochem. Biophys. Res. Commun., 2015, 466, 350-355., Carbyhydrate Research (2012) vol. 364, pp. 41-48, and the like. The method for cleavage from glycoproteins is also not particularly limited, but examples thereof include chemical, biological, or enzymatic degradation of glycoproteins, or a combination thereof. Furthermore, the method using chemical synthesis and the method for cleavage from glycoproteins may be used in combination. As an example of a specific method, glycoamino acids may be obtained by degrading glycoproteins with proteases, and glycooligopeptides may be obtained by extending the peptide chain from glycoamino acids by a chemical synthesis method.

In the present embodiment, the mixture containing glycoamino acids and one or more types of glycooligopeptides is not particularly limited as long as it contains glycoamino acids and one or more types of glycooligopeptides; however, the mixture may also contain components other than the glycoamino acids and the one or more types of glycooligopeptides.

The components other than the glycoamino acids and the one or more types of glycooligopeptides are not particularly limited, but include, when the glycopeptides are obtained by chemical synthesis, the reagents etc. used in the chemical synthesis. When the glycopeptides are obtained by cutting from an antibody, the components include peptide fragments derived from the antibody, glycans, amino acids, and glycopeptides other than the glycoamino acids and glycooligopeptides. In addition, the components necessary for the denaturation and decomposition of the antibody may also be contained.

### (Separation)

In the present embodiment, a mixture containing glycoamino acids and one or more types of glycooligopeptides can be passed through an anion-exchange resin to thereby separate the glycoamino acids and the one or more types of glycooligopeptides from the mixture.

The separation of the glycoamino acids and the one or more types of glycooligopeptides contained in the mixture means that the glycoamino acids and the one or more types of glycooligopeptides are sequentially eluted in the eluate from the anion-exchange resin, whereby, for example, the glycoamino acids and the glycooligopeptides can be separated and collected as individual fractions.

The order in which the glycoamino acids and the glycooligopeptides are eluted is not particularly limited.

The glycoamino acids fraction and the glycooligopeptide fraction may contain glycoamino acids and glycooligopeptides having different glycan structures, respectively.

### (Anion-Exchange Resin)

The anion-exchange resin is not particularly limited, but examples thereof include strong anion-exchange resins, weak anion-exchange resins, and the like. From the viewpoint of more efficiently separating the glycoamino acids and the one or more types of glycooligopeptides, weak anion-exchange resins are preferred.

The base material of the anion-exchange resin is not particularly limited, but examples thereof include styrene, divinylbenzene, cellulose, agarose, and the like. From the viewpoint of more efficiently separating the glycoamino acids and the one or more types of glycooligopeptides, cellulose is preferred.

Examples of strong anion-exchange resins include those in which a quaternary amino group is introduced into base materials.

Examples of weak anion-exchange resins include those in which a tertiary amino group, a diethylaminoethyl group, a carboxylmethyl group, a polyamine, or the like is introduced into base materials.

The method for separating, from a mixture containing glycoamino acids and one or more types of glycooligopeptides, the glycoamino acids and the one or more types of glycooligopeptides by passing the mixture through an anion-exchange resin is not particularly limited, but examples thereof include anion-exchange chromatography.

A conventionally known buffer solution can be used as the mobile phase used in anion-exchange chromatography.

The solvent used in the buffer solution is not particularly limited, and examples thereof include water, acetonitrile, alcohol, and the like. A single solvent may be used, or a solvent mixture of two or more solvents may be used. From the viewpoint of more efficiently separating the glycoamino acids and the one or more types of glycooligopeptides, a solvent mixture of water and acetonitrile is preferred.

The component of the buffer solution is not particularly limited, but examples thereof include components having buffering capacity, such as acetic acid, phosphoric acid, citric acid, alkylamine, ammonium, trishydroxymethylaminomethane, pyridine, and triethylamine. A single component may be used, or two or more components may be used. From the viewpoint of more efficiently separating the glycoamino acids and the one or more types of glycooligopeptides, acetic acid and triethylamine are preferred.

The pH of the mobile phase is not particularly limited; however, from the viewpoint of more efficiently separating the glycoamino acids and the one or more types of glycooligopeptides, the pH is preferably in the neutral to basic range, and more preferably in the range of 7 to 10.

In the present embodiment, when anion-exchange chromatography is used, elution may be performed with a gradient in the mobile phase. For example, when a gradient is applied to the mobile phase, elution may be performed so that the gradient is applied at a pH in the range of 7 to 10.

In order to separate, from a mixture containing glycoamino acids and one or more types of glycooligopeptides, the glycoamino acids and the glycooligopeptides, the number of times the mixture is passed through an anion-exchange resin is not limited to once, and may be multiple times. Furthermore, before passing the mixture containing glycoamino acids and one or more types of glycooligopeptides through an anion-exchange resin, the mixture may be roughly purified.

### Examples

The present embodiment will be described in more detail below with reference to Examples and Comparative Examples; however, the present embodiment is not limited to only these Examples. The measurement method used in the present embodiment is as described below.

### [Mass Spectrometry]

MALDI-8030, manufactured by Shimadzu Corporation
Mode: Linear Positive

### <Example 1>

The method described in the specification was used to obtain glycopeptides in which the number of amino acids in peptides was 1 to 3, and to which a glycan of 8 to 12 neutral monosaccharides was linked. Thereafter, a mixture containing the glycopeptides in which the number of amino acids in peptides was1 to 3, and to which a glycan of 8 to 12 neutral monosaccharides was linked, was dissolved in 10% acetonitrile (pH = 9.5, prepared with triethylamine), and mass spectrometry was performed by MALDI-TOF MS (Figure 1). Table 1 shows the molecular weights of glycoamino acids, glycodipeptides, and glycotripeptides contained in the mixture actually measured by mass spectrometry. In the table, "Content < analytical sensitivity" means that the content was lower than the analytical sensitivity of mass spectrometry. From the actual values shown in Tables 2 and 3 as the results of mass spectrometry after separation, it can be understood that glycopeptides, which had been below the analytical sensitivity before separation, were present in the mixture before separation.

**[Table 1]**

| | High-mannose, 12 monosacchari des | High-mannose, 11 monosacchari des | High-mannose, 10 monosacchari des | High-mannose, 9 monosacchar ides | High-mannose, 8 monosacchar ides |
|---|---|---|---|---|---|
| Glycoamin o acid | Peak overlap with high-mannose, 11 monosacchari des, glycotripeptid e | Peak overlap with high-mannose, 10 monosacchari des, glycotripeptid e | Peak overlap with high-mannose, 9 monosacchari des, glycotripeptid e | Content < analytical sensitivity | Content < analytical sensitivity |
| Glycodipep tide | 2237.898 | 2076.577 | 1913.702 | Content < analytical sensitivity | Content < analytical sensitivity |
| Glycotripep tide | 2338.919 | 2176.833 | 2015.556 | 1852.658 | Content < analytical sensitivity |

| | Complex-type, 11 monosacchari des | Complex-type, 10 monosacchari des | Complex-type, 9 monosacchari des |
|---|---|---|---|
| Glycoamin o acid | 2126.545 | 1964.658 | Content < analytical sensitivity |

About 100 mg of the above mixture was applied to about 300 mL of an anion-exchange support (Cellfine A-500, manufactured by JNC Corporation) equilibrated with 10% acetonitrile (pH = 9.5, prepared with triethylamine), and anion-exchange purification was performed under the following conditions.
- Solvent A = 10% acetonitrile (pH = 9.5, prepared with triethylamine)
- Solvent B = 3% acetic acid (pH = 7.5, prepared with triethylamine) : acetonitrile = 9:1
- Flow rate: Natural drop
- Gradient conditions
   (1) 600 mL of 100% solvent A
   (2) 600 mL of solvent A:solvent B = 99:1
      30 mL of each of (1) and (2) was collected, and each fraction was analyzed by MALDI-TOF MS.

After applying the sample, the presence of glycopeptides in which the number of amino acids was 1, and to which a glycan of 8 to 12 neutral monosaccharides or a complex-type glycan of 9 to 11 monosaccharides was linked, was confirmed in a fraction eluted in about 300 to 500 mL (Figure 2), and the presence of glycopeptides in which the number of amino acids was 2 or 3, and to which a glycan of 8 to 12 neutral monosaccharides was linked, was confirmed in a fraction eluted in about 800 to 1000 mL (Figure 3).

Table 2 shows the molecular weights of glycoamino acids contained in the fraction eluted in about 300 to 500 mL actually measured by mass spectrometry. In the table, "Content < analytical sensitivity" means that the content was lower than the analytical sensitivity of mass spectrometry.

**[Table 2]**

| | High-mannose, 12 monosacchar ides | High-mannose, 11 monosacchar ides | High-mannose, 10 monosacchar ides | High-mannose, 9 monosacchar ides | High-mannose, 8 monosacchar ides |
|---|---|---|---|---|---|
| Glycoamino acid | 2181.570 | 2020.202 | Content < analytical sensitivity | Content < analytical sensitivity | 1533.032 |

| | Complex-type, 11 monosacchar ides | Complex-type, 10 monosacchar ides | Complex-type, 9 monosacchar ides |
|---|---|---|---|
| Glycoamino acid | 2126.419 | 1964.399 | 1802.457 |

Table 3 shows the molecular weights of glycodipeptides and glycotripeptides contained in the fraction eluted in about 800 to 1000 mL actually measured by mass spectrometry.

**[Table 3]**

| | High-mannose, 12 monosacchar ides | High-mannose, 11 monosacchar ides | High-mannose, 10 monosacchar ides | High-mannose, 9 monosacchar ides | High-mannose, 8 monosacchar ides |
|---|---|---|---|---|---|
| Glycodipep tide | 2238.582 | 2077.092 | 1914.144 | 1751.591 | 1590.816 |
| Glycotripep tide | 2339.238 | 2177.279 | 2015.284 | 1853.264 | 1691.077 |

As shown in Figure 1, before the mixture was passed through an anion-exchange resin, peaks were detected indicating glycopeptides in which the number of amino acids in peptides was 1 to 3 amino acids, and to which a glycan of 8 to 12 neutral monosaccharides or a complex-type glycan of 9 to 11 monosaccharides was linked. On the other hand, when the fractions obtained by passing the mixture through an anion-exchange resin were subjected to mass spectrometry, it was revealed that they were separated into a fraction containing glycopeptides in which the number of amino acids was 1, and to which a glycan of 8 to 12 neutral monosaccharides or a complex-type glycan of 9 to 11 monosaccharides was linked, and a fraction containing glycopeptides in which the number of amino acids was 2 or 3, and to which a glycan of 8 to 12 neutral monosaccharides was linked.

### [Molecular Weight of Each Glycopeptide]

High-mannose-type, 12 monosaccharides, glycotripeptide (Asn-Gly-Thr): 2339.80 [M+Na], high-mannose-type, 11 monosaccharides, glycotripeptide (Asn-Gly-Thr): 2177.75 [M+Na], high-mannose-type, 10 monosaccharides, glycotripeptide (Asn-Gly-Thr): 2015.69 [M+Na], high-mannose-type, 9 monosaccharides, glycotripeptide (Asn-Gly-Thr): 1853.64 [M+Na], high-mannose-type, 8 monosaccharides, glycotripeptide (Asn-Gly-Thr): 1691.59 [M+Na], high-mannose-type, 12 monosaccharides, glycodipeptide (Asn-Gly): 2237.77 [M+Na], high-mannose-type, 11 monosaccharides, glycodipeptide (Asn-Gly): 2075.71 [M+Na], high-mannose-type, 10 monosaccharides, glycodipeptide (Asn-Gly): 1913.66 [M+Na], high-mannose-type, 9 monosaccharides, glycodipeptide (Asn-Gly): 1751.61 [M+Na], high-mannose-type, 8 monosaccharides, glycodipeptide (Asn-Gly): 1590.54 [M+Na], high-mannose-type, 12 monosaccharides, glyco-Asn: 2181.73 [M+Na], high-mannose-type, 11 monosaccharides, Asn: 2019.68 [M+Na], high-mannose-type, 10 monosaccharides, Asn: 1867.62 [M+Na], high-mannose-type, 9 monosaccharides, Asn: 1695.57 [M+Na], high-mannose-type, 8 monosaccharides, Asn: 1533.52 [M+Na], complex-type, 11 monosaccharides, Asn: 2126.76 [M+Na], complex-type, 10 monosaccharides, Asn: 1964.71 [M+Na], complex-type, 9 monosaccharides, Asn: 1802.66 [M+Na]

## Claims

1. A method for separating, from a mixture containing glycoamino acids and one or more types of glycooligopeptides, the glycoamino acids and the glycooligopeptides by passing the mixture through an anion-exchange resin.

2. The method according to claim 1, wherein the glycooligopeptides are glycodipeptides and/or glycotripeptides.

3. The method according to claim 1, wherein the glycoamino acids and the glycooligopeptides are each derived from an antibody.

4. The method according to claim 1, wherein the glycoamino acids and the glycooligopeptides each have a glycan of 8 to 12 monosaccharides.

5. The method according to claim 1, wherein the glycoamino acids and the glycooligopeptides each have a glycan of only neutral monosaccharides.
